# EUROPEAN PATENT APPLICATION

(11) **EP 2 495 566 A1**
(43) Date of publication of application: **05.09.2012**
(21) Application number: 11001695.3
(22) Date of filing: 01.03.2011
(51) Int. Cl.: G01N 33/564, G01N 33/68

(54) **Il-22 as a prognostic and diagnostic marker in rheumatoid arthritis**

(71) Applicant: Ludwig-Maximilians-Universität München, 80539 München (DE)
(72) Inventor: Schulze-Koops, Hendrik, Prof. Dr., 80336 München (DE); Skapenko, Alla, Dr. rer.nat., 80336 München (DE); Leipe, Jan, Dr. med., 80336 München (DE)
(74) Representative: Engelhard, Markus

(57) **Abstract**

The present invention relates to interleukin-22 (IL-22) for use as a prognostic marker for bone erosion associated with rheumatoid arthritis (RA) and for use as a diagnostic marker for the progression of RA. It further relates to a method of prognosing the development of bone erosion associated with rheumatoid arthritis (RA), to a method of diagnosing the progression of RA and to the use of an IL-22-specific test kit for prognosing the development of bone erosion or diagnosing the progression of rheumatoid arthritis in a subject suffering from RA.

## Description

The present invention relates to interleukin-22 (IL-22) for use as a prognostic marker for bone erosion associated with rheumatoid arthritis (RA) and for use as a diagnostic marker for the progression of RA. It further relates to a method of prognosing the development of bone erosion associated with rheumatoid arthritis (RA), to a method of diagnosing the progression of RA and to the use of an IL-22-specific test kit for prognosing the development of bone erosion or diagnosing the progression of rheumatoid arthritis in a subject suffering from RA.

Rheumatoid arthritis (RA) is an autoimmune disease characterized inter alia by synovitis (i.e. the inflammation of the synovial membrane) and the development of bone erosions, ultimately leading to joint destruction in the course of the disease. About 1% of the world's population is afflicted by RA, women three times more often than men. Onset is most frequently between the ages of 40 and 50, but people of any age can be affected.

The term "bone erosion" describes the loss of mineralized tissue at juxta-articular sites, which is commonly associated with a break in the cortical lining. Determination of bone erosion is almost exclusively based on radiographic findings because direct assessment of these lesions through biopsy is only rarely practical. Other ways of diagnosing bone erosion include magnetic resonance imaging (MRI), computer tomography (CT) and ultrasonography.

Nowadays, there is a trend toward early diagnosis and treatment of RA, i.e. before joint destruction occurs. Bone erosion and destruction of joints viewed in radiological images was a significant point of the American College of Rheumatology (ACR) Rheumatoid Arthritis Classification Criteria of 1987 (1). According to the new classification criteria jointly published by the ACR and the European League against Rheumatism (EULAR) (2), this criterion is no longer regarded to be relevant, as this is just the type of damage that treatment of RA is meant to avoid.

For the diagnosis of RA, the "new" classification criteria cover the following four areas:
- joint involvement - depending on the type and number of joints;
   - serological parameters - including the rheumatoid factor (RF) as well as "anti-citrullinated protein antibody" (ACPA or anti-CCP for "anti-cyclic citrullinated peptide");
   - acute phase reactants: e.g. elevated erythrocyte sedimentation rate (ESR) or elevated CRP value (c-reactive protein); and
   - duration of arthritis.

Besides the above criteria, further criteria are used for the diagnosis of RA, e.g. in the common clinical practice:
- two or more swollen joints;
   - morning stiffness lasting more than one hour for at least six weeks; and
   - the detection of rheumatoid factor or auto-antibodies against ACPA such as auto-antibodies to mutated citrullinated vimentin can confirm the suspicion of rheumatoid arthritis. A negative autoantibody result does not exclude a diagnosis of RA.

It is an object of the present invention to provide novel molecular markers of RA and its symptoms, such as bone erosion, and particularly markers, which help to predict clinical outcome in very early RA. It is a further object of the present invention, to provide molecular markers which allow to diagnose/monitor the progression of RA.

The objects of the present invention are solved by interleukin-22 (IL-22) for use as a prognostic marker for bone erosion associated with rheumatoid arthritis.

The term "prognostic marker" (or prognostic indicator) refers to the fact that an increased concentration of IL-22 in an early stage of RA indicates a predisposition (or increased risk) for developing bone erosion.

Interleukin-22 (IL-22) is a pro-inflammatory cytokine primarily expressed by T helper 17 (Th17) cells) (3). However, in humans significant IL-22 production was also demonstrated in so called "Th22" cells - a recently described CD4 T cell population that is negative for IL-17A and IFN-γ - and in Th1 cells (4). In addition to these pure subsets of cells, cells with a mixed phenotype capable of producing all of IL-22, IL-17 and IFN-γ can be detected in the human blood (5).

In one embodiment, a concentration of IL-22 measured in the early stages of rheumatoid arthritis, which is increased as compared to the concentration of IL-22 in healthy controls, indicates an increased risk of developing bone erosion within 24 months, preferably within 12 months, more preferably within 10 months, even more preferably within 8 months after the initial (i.e. first) clinical symptoms of rheumatoid arthritis.

The term "early stages of rheumatoid arthritis", as used herein, refers to the first 6 months, preferably the first 5 months, more preferably the first 4 months, most preferably the first 3 months (referred to as "very early") after the initial clinical symptoms of rheumatoid arthritis. Accordingly, in one embodiment, said increased concentration of IL-22 is measured within the first 6 months, preferably within the first 5 months, more preferably within the first 4 months, most preferably within the first 3 months after the initial clinical symptoms of rheumatoid arthritis.

"Initial clinical symptoms" of RA are known to a person skilled in the art and include two or more swollen joints, preferably of the hands or feet, morning stiffness lasting more than one hour for at least six weeks, the presence of rheumatoid factor (RF) and/or ACPA/CCP and serological signs of systemic inflammation (such as elevated CRP or ESR). Detecting the presence of ACPA/CCP is particularly suitable for diagnosing the disease in an early state.

The "concentration of IL-22 in healthy controls" can also be referred to as "normal concentration of IL-22". "Healthy controls" (HC) are age-matched individuals without RA or any other known condition potentially affecting the concentration of IL-22.

Means and ways to measure the concentration of IL-22 are known to a person skilled in the art. Generally, the concentration of IL-22 can be determined by detecting the IL-22 protein (e.g. by detecting the binding of an antibody against the IL-22 protein), but also by detecting the amount of IL-22 mRNA transcripts (e.g. by detecting hybridization to a gene transcript of the IL-22 gene or by PCR methods, such as Real-time PCR), for instance in the tissue.

Antibody-based detection methods are well known in the art and include ELISA, radioimmunoassays, immunoblots (e.g. Western blots), flow cytometry, immunofluorescence, immunoprecipitation and other related techniques. Antibodies may be modified with detectable labels, including ligand groups (e.g. biotin), fluorophores and chromophores, radioisotopes, electron-dense reagents or enzymes, wherein such enzymes are detected by their activity.

In one embodiment, said concentration of IL-22 is increased by at least 75%, preferably by at least 100%, most preferably by at least 125%, as compared to the concentration of IL-22 in healthy controls.

In one embodiment, RA patients with increased IL-22 concentration in the early stages of RA have a relative risk of developing bone erosion which is at least 20, preferably at least 25, more preferably at least 30, one year after the initial clinical symptoms of RA, and/or a relative risk which is at least 25, preferably at least 30, more preferably at least 35, two years after the initial clinical symptoms of RA. The term "relative risk" refers to the ratio of the probability of the development of bone erosion versus the probability of the development of bone erosion in RA patients showing no increased IL-22 concentration in the early stages of RA. A relative risk X means that patients with increased IL-22 concentration in the early stages of RA are X-times as likely to develop bone erosion as RA patients showing no increased IL-22 concentration in the early stages of RA.

The objects are also solved by a method of prognosing the development of bone erosion in a subject suffering from rheumatoid arthritis, said method comprising the steps of:
a) measuring the concentration of IL-22 in a sample derived from said subject; and
b) comparing said concentration of IL-22 with the concentration of IL-22 in healthy controls;
wherein said method is performed within the first 6 months, preferably within the first 5 months, more preferably within the first 4 months, most preferably within the first 3 months after the initial (i.e. first) clinical symptoms of rheumatoid arthritis, and
wherein an increased concentration of IL-22 as compared to the concentration of IL-22 in healthy controls indicates an increased risk of developing bone erosion within 24 months, preferably within 12 months, more preferably within 10 months, even more preferably within 8 months after the initial clinical symptoms of rheumatoid arthritis.

In one embodiment, said sample is selected from the group consisting of a blood sample, a serum sample and a plasma sample. Preferably, said sample is a serum sample.

In one embodiment, the step of deriving the sample from said subject is not part of the method of prognosing the development of bone erosion.

In one embodiment, the step of measuring the concentration of IL-22 is an in-vitro-step, i.e. it is performed in-vitro.

In one embodiment, said concentration of IL-22 is increased by at least 75%, preferably by at least 100%, most preferably by at least 125%, as compared to the concentration of IL-22 in healthy controls.

In one embodiment, RA patients with increased IL-22 concentration in the early stages of RA have a relative risk of developing bone erosion which is at least 20, preferably at least 25, more preferably at least 30, one year after the initial clinical symptoms of RA, and/or a relative risk which is at least 25, preferably at least 30, more preferably at least 35, two years after the initial clinical symptoms of RA. The term "relative risk" refers to the ratio of the probability of the development of bone erosion versus the probability of the development of bone erosion in RA patients showing no increased IL-22 concentration in the early stages of RA. A relative risk of X means that patients with increased IL-22 concentration in the early stages of RA are X-times as likely to develop bone erosion as RA patients showing no increased IL-22 concentration in the early stages of RA.

The objects of the present invention are also solved by interleukin-22 (IL-22) for use as a diagnostic marker for the progression of rheumatoid arthritis.

The term "diagnostic marker" (or diagnostic indicator) refers to the fact that the concentration of IL-22 correlates with the severity of several symptoms of RA, including synovitis and the number of tender and swollen joints in patients with increased concentration of IL-22 in an early stage of RA, and thus can be used as a direct indicator/marker of the progression of RA.

In one embodiment, the concentration of IL-22 correlates with the severity of synovitis and/or the number of tender and swollen joints, such that a higher concentration of IL-22 corresponds to more severe synovitis and/or a higher number of tender and swollen joints.

The objects of the present invention are further solved by a method of diagnosing the progression of rheumatoid arthritis in a subject, said method comprising the step of measuring the concentration of IL-22 in a sample derived from said subject, wherein the concentration of IL-22 correlates with the severity of synovitis and/or the number of tender and swollen joints, such that a higher concentration of IL-22 corresponds to more severe synovitis and/or a higher number of tender and swollen joints.

In one embodiment the step of deriving the sample from said subject is not part of the method of diagnosing the progression of rheumatoid arthritis. In one embodiment, the step of measuring the concentration of IL-22 is an in-vitro-step, i.e. it is performed in-vitro.

In one embodiment, said sample is selected from the group consisting of a blood sample, a serum sample and a plasma sample. Preferably, said sample is a serum sample.

In one embodiment, said method further comprises the step of comparing the measured concentration of IL-22 with a standardized chart showing particular stages of RA as a function of IL-22 concentration.

In preferred embodiments of the methods according to the present invention, IL-22 is the only marker that is used for prognosing the development of bone erosion in a subject suffering from rheumatoid arthritis or for diagnosing the progression of rheumatoid arthritis.

The objects of the present invention are further solved by the use of an IL-22-specific test kit for prognosing the development of bone erosion or diagnosing the progression of rheumatoid arthritis in a subject suffering from rheumatoid arthritis.

IL-22-specific test kits, which allow to measure the concentration of IL-22 in a sample derived from a subject suffering from rheumatoid arthritis, are known to a person skilled in the art and are, for example, based on antibody-based detection methods, such as ELISA.

The inventors have surprisingly found that interleukin-22 (IL-22) is involved in the pathophysiology of rheumatoid arthritis (RA) and can be used to predict the clinical outcome in very early RA. They have found that an increased concentration of IL-22 in an early stage of RA indicates a predisposition (or increased risk) to develop bone erosion at a later stage. Furthermore, they have found that in RA patients having increased IL-22 concentration at early stages of RA, IL-22 concentration correlates with the severity of synovitis and the number of tender and swollen joints, which allows to monitor the progression of RA by simply measuring the concentration of IL-22.

### FIGURES

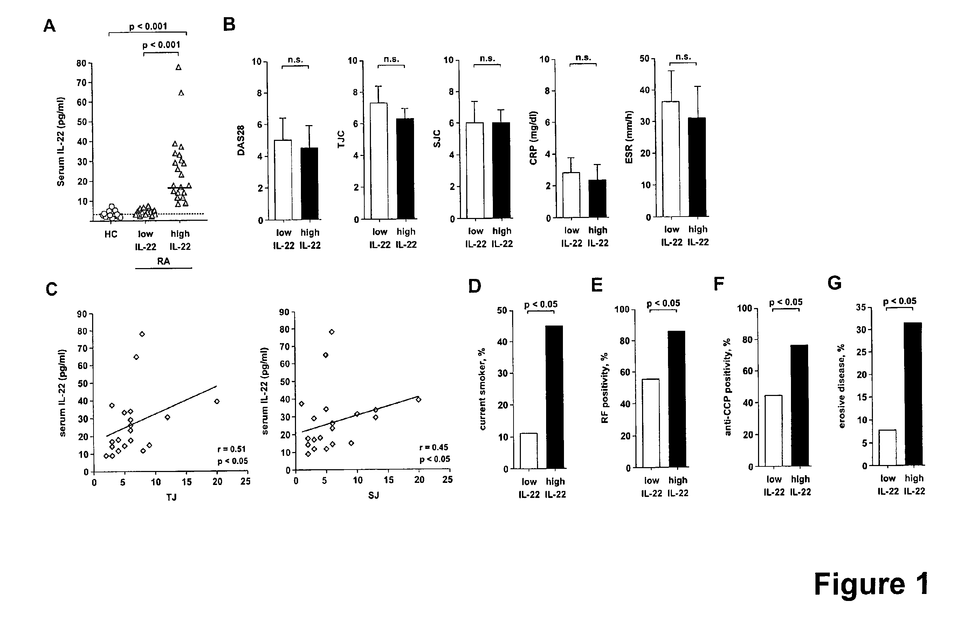

**Figure 1** documents that increased levels of IL-22 are associated with disease outcome in early RA. **(A)** Serum IL-22 concentrations of HC (n=15), and cohorts of RA patients characterized by low IL-22 levels (n=18,"IL-22 low group")or high IL-22 levels (n=21,"IL-22 high group"). The horizontal lines show the mean, the broken horizontal line indicates the detection limit of the assay. **(B)** Disease activity score in 28 joints (DAS28), tender joint count (TJC), swollen joint count (SJC), C-reactive protein level (CRP), and erythrocyte sedimentation rate (ESR) of the patients in the "IL-22 low group" and "IL-22 high group", respectively. **(C)** Spearman rank correlation of IL-22 serum levels with TJC and SJC in the "IL-22 high group". r-Spearman's rank correlation coefficient. **(D)** Comparison of the proportion of patients in the "IL-22 low group" and "IL-22 high group" with regard to current smoking, **(E)** rheumatoid factor (RF) positivity, **(F)** anti-CCP positivity, and **(G)** the development of an erosive disease. Bars show the mean. P values were determined by Student' t-test.

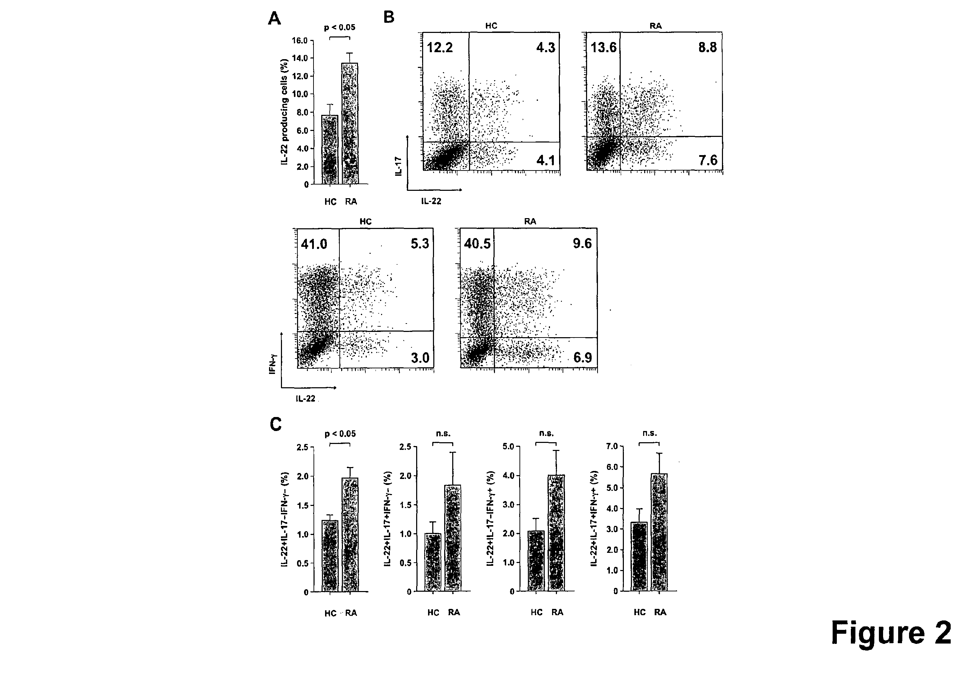

**Figure 2** documents an increase in IL-22 producing cells in RA. **(A)** Boolean gating analysis demonstrating mean proportions of IL-22 producing cell populations based on cytokine production of IL-22, and IL-17 and IFN-γ by memory CD4 T cells after 7 days of expansion, stimulation with PMA and Ionomycin, followed by intracellular cytokine staining. Data are shown as mean percentages of 5 HC and 4 RA patients, calculated as the proportion of the respective population within IL-22 producing cells. **(B)** Frequencies of IL-22 producing cells within the memory CD4 T cells, and **(C)** frequencies of the IL-22+IL-17-IFN-γ- population, the IL-22+IL-17+IFN-γ- population, the IL-22+IL-17-IFN-γ+ population, and the IL-22+IL-17+IFN-γ+ population in RA patients compared to HC. Bars show the mean and SEM of 5 HC and 4 RA patients. P values were determined by Student' t-test.

### EXAMPLES

### 1. Material and Methods

### 1.1 Study population

Peripheral blood (PB) was obtained from 39 patients who fulfilled the American College of Rheumatology revised criteria for a diagnosis of RA (1) **(Table 1).** The patients had active disease as defined by a Disease Activity Score in 28 joints (DAS 28) of ≥ 3.2. All patients had a mean duration of less than 3 months since their initial clinical symptoms (referred to as very early), and had never been treated with glucocorticoids or disease modifying antirheumatic drugs (DMARDs) (referred to as treatment-naive patients). Demographic and clinical parameters such as age, gender, disease duration, NSAID use, DAS28 values, erythrocyte sedimentation rate (ESR) and levels of C-reactive protein (CRP), rheumatoid factor (RF), and anti-cyclic citrullinated peptide (CCP) were recorded at the time of blood sampling. For control, age-matched healthy individuals were analyzed **(Table 1).** Written informed consent was provided by all patients and healthy donors. The study was approved by the ethics committee of the University of Munich.

**Table 1. Study population^{a}**

| | Healthy controls | RA (n=39) | RA, IL-22 Low | RA, IL-22 High |
|---|---|---|---|---|
| | (n=14) | | Group (n=18)^{b} | Group (n=18)^{c} |
| Age, yrs | 56.1 ± 4.9 | 53.8 ± 10.8 | 55.2 ± 10.4 | 52.7 ± 11.2 |
| Female:male ratio, n | 9:5 | 28:1 | 15:3 | 14:7 |
| Disease duration, months | n.a.^{d} | 4.3 ± 3.8 | 4.3 ± 4.2 | 3.9 ± 3.2 |
| NSAID use,% | n.a. | 69.2 | 66.6 | 71.4 |

| | | | | |
|---|---|---|---|---|
| ^{a} data are shown as mean ± SD or absolute numbers. RA = rheumatoid arthritis ^{b} cohorts of RA patients characterized by low IL-22 levels ("IL-22 Low Group")^{c} cohorts of RA patients characterized by high IL-22 levels ("IL-22 High Group") ^{d} not applicable | | | | |

### 1.2 Radiographical evaluation

Radiographs of hands and feet were performed within two years after disease onset. The radiographs were evaluated for the presence of erosions by an expert blinded to clinical and other imaging data. The patients who developed erosions within the first two years after onset of the disease were classified as having "erosive disease".

### 1.3 Cell purification

Mononuclear cells from PB were isolated by Ficoll density-gradient centrifugation (Lymphoflot, Biotest AG, Dreieich, Germany). Untouched memory (CD45RO) CD4 T cells were isolated by negative selection using magnetic beads (Miltenyi Biotech, Bergisch Gladbach, Germany). Purity was routinely controlled by flow cytometry. Memory CD4 T cells, were ≥ 95% positive for CD3/4, and ≥ 95% of memory cells stained brightly for CD45RO.

### 1.4 Cell culture

Cell cultures were performed in RPMI 1640 medium supplemented with penicillin G (50 U/ml), 50 µg/ml streptomycin, 2 mM L-glutamine (all from Gibco/Invitrogen, Carlsbad, CA), 10 IU/ml recombinant human IL-2 (Proleukin; Chiron, Emeryville, CA), and 10% normal human serum (NHS) at 37°C in a humidified atmosphere containing 5% CO₂. To improve detectability of IL-22 producing populations, memory CD4 T cells (0,5 × 10⁶ cells/ml) were activated in 48-well plates under non-priming condition for 7 days by plate-bound anti-CD3 (1 µg/ml; OKT3, American Type Culture Collection, Manassas, VA) and soluble anti-CD28 (1 µg/ml; 28.2; BD Bioscience, San Jose, CA), and rested for 2 days. For intracellular cytokine staining, cells were re-stimulated for 5h with 1 mM ionomycin (Calbiochem, San Diego, CA) and 20 ng/ml PMA in the presence of 2 µM monensin (both from Sigma-Aldrich, St. Louis, MO).

### 1.5 Flow cytometry

For analysis of surface phenotypes, cells were washed with 2% FCS/PBS, stained with directly fluorochrome-labeled mAbs against different surface molecules as indicated, and analyzed on a Cytomics FC500 flow cytometer with CXP software (Beckman Coulter, Fullerton, CA). The antibodies used were as follows: FITC-labeled anti-CD3, PE-labeled anti-CD4, FITC-labeled anti-CD27, PE-labeled anti-CD45RO (all from BD Biosciences). For intracellular cytokine staining, re-stimulated cells were fixed in 3% paraformaldehyde (Merck, Darmstadt, Germany) and permeabilized with 0.1% saponin (Sigma-Aldrich) in PBS containing 2% FCS (Gibco/Invitrogen), and the nonspecific binding sites were blocked with 4% rat and mouse serum (both from Sigma-Aldrich). In the next step, cells were incubated with FITC-labeled anti-IFN-γ, PE-labeled anti-IL-22 (both BD Biosciences), and allophycocyanin (APC)-labeled anti-IL-17 (eBiosciences, San Diego, CA) for 30 minutes at 4°C. After washing with 0.1% saponin in 2% FCS/PBS, cells were resuspended in 2% FCS/PBS and analyzed for cytoplasmic cytokines by 3-color flow cytometry. The Th profile of the cells was defined by their ability to express IL-17 but not IFN-γ (Th17 cells), IFN-γ but not IL-17 (Th1 cells) or IL-22 but not IL-17 or IFN-γ (Th22 cells).

### 1.6 Enzyme-linked immunosorbent assay (ELISA)

Levels of IL-22 were assessed using ELISA kits according to the manufacturer's instructions (R&D Systems) in the serum. The normal range of IL-22 serum concentration is defined by the mean levels of age-matched healthy individuals ± three standard deviations.

### 1.7 Statistical analysis

Differences between the cohorts were determined by Student's 2-tailed t-tests. Correlations were analyzed using Spearman's rank correlation coefficients. P values less than 0.05 were considered statistically significant. All analyses were performed using Prism 5.0 software (GraphPad software, San Diego, CA, USA).

### 2. Results

### 2.1 IL-22 baseline levels in RA patients are associated with clinical outcome

To investigate the role of IL-22 in the systemic autoimmune disease rheumatoid arthritis, first IL-22 levels were first determined in the peripheral blood of a cohort of RA patients with very early, active disease. These patients had never been treated with glucocorticoids or disease modifying antirheumatic drugs (DMARDs) to rule out possible influences of immunosuppressive medication on cytokine expression **(Table 1).**

Serum IL-22 levels were generally higher in RA patients compared to HC **(****Figure 1A****).** Of interest, within the RA patients two groups were identified: a first group with IL-22 levels within the normal range ("low IL-22 group") and a second group with markedly elevated IL-22 serum levels ("high IL-22 group") **(****Figure 1A****).** Both groups did not differ significantly at baseline with regard to parameters of disease activity, e.g. disease activity score (DAS28), tender joint count (TJC), swollen joint count (SJC), C-reactive protein, and erythrocyte sedimentation rate (ESR) **(****Figure 1B****).** However, in the "high IL-22 group" serum levels of IL-22 correlated with the number of tender and swollen joints at baseline as well as with the severity of synovitis based on the correlation of serum parameters with the clinical data of individual patients. Moreover, in the "high IL-22 group" of patients the proportion of smokers, the presence of rheumatoid factor (RF) and antibodies to citrullinated peptides (anti-CCP) were significantly higher compared to the "low IL-22 group" **(Table 2,** **Figure 1D-F).**

**Table 2. Population of RA patients^{a}**

| | IL-22 | | |
|---|---|---|---|
| | serum level | | |
| | Low pos.RA (n=18) | High pos.RA (n=21) | P-value |
| IL-22 serumlevel, pg/ml | 4.5 ± 1.6 | 26.6 ± 17.8 | p < 0.001 |
| IL-17 serumlevel, pg/ml | 2.2 ± 1.5 | 11.9 ± 18.3 | p < 0.05 |
| Age, yrs | 55.2 ± 10.4 | 52.7 ± 11.2 | n.s. |
| Female:maleratio, n | 15:3 | 14:7 | n.s. |
| Disease dura-tion, months | 4.3 ± 4.2 | 3.9 ± 3.2 | n.s. |
| NSAID use, % | 66.6 | 71.4 | n. s. |
| RF positive, % | 55.5 | 85.7 | p < 0.05 |
| Anti-CCP posi-tive, % | 44.4 | 76.2 | p < 0.05 |
| DAS28 | 5.0 ± 1.4 | 4.5±1.4 | n.s. |
| TJC, n | 7.3±5.7 | 6.3±3.9 | n.s. |
| SJC, n | -6.0 ± 5.7 | 6.0 ± 4.7 | n. s. |
| CRP, mg/dl | 2.9±4.0 | 2.4±4.6 | n.s. |
| ESR, mm/hour | 36.9±35.2 | 31.6±34.6 | n.s. |
| Current smoker,% | 11.1 | 45.0 | p < 0.05 |
| Erosive disease,% | 5.5 | 28.6 | p < 0.05 |

| | | | |
|---|---|---|---|
| ^{a} data are shown as mean ± SD or absolute numbers | | | |

Patients having high baseline IL-22 serum levels and being followed up for radiographic progression, significantly more frequently developed bone erosions within the first 2 years **(****Figure 1G****)** and as early as 6 months.

Generally, bone erosion appears to occur at early stages of R.A - however, if bone erosion does not occur at such early stages, an increased concentration of IL-22 indicates an increased risk of a subsequent development of bone erosion.

Together, the data suggest that although the patients with high IL-22 levels have similar baseline disease activity parameters in comparison to patients with low IL-22 levels, they are more likely to develop bone erosions.

### 2.2 Increased IL-22 producing cells in RA

To investigate the cellular source of elevated IL-22 levels in RA, the frequencies of different memory CD4 T cell populations producing IL-22 were assessed in RA patients. Overall, memory CD4 T cells of the RA patients contained substantially more IL-22 producing cells as compared to control cells **(****Figure 2A****).** Further analysis revealed higher frequencies of cells producing IL-22 either alone or in combination with IL-17, or IFN-γ respectively **(****Figure 2B****).** In the Boolean gating analysis of the memory CD4 T cells from RA patients and controls that were stained for IL-22, IL-17 and IFN-γ significantly higher frequencies of IL-22+IL-17-IFN-γ- cells ("Th22") were detected **(****Figure 2C****).** Similar, the frequencies of IL-22+IL-17+IFN-γ- cells, of IL-22+IL-17-IFN-γ+ cells, and of IL-22+IL-17+ IFN-γ+ cells tended to be higher in RA patients **(****Figure 2C****).** Together, the data indicate an enforced expression of IL-22 by memory CD4 T cells as a potential source of the increased IL-22 levels observed in RA patients.

### REFERENCES

1. Arnett FC, Edworthy SM, Bloch DA, McShane DJ, Fries JF, Cooper NS, et al. The American Rheumatism Association 1987 revised criteria for the classification of rheumatoid arthritis. Arthritis Rheum. 1988. 31: 315-24.
2. Aletaha D, Neogi T, Silman AJ, et al. 2010 Rheumatoid Arthritis Classification Criteria: An American College of Rheumatology/European League Against Rheumatism collaborative initiative. Ann. Rheum. Dis. 2010. 69 (9):1580-8.
3. Liang SC, Tan XY, Luxenberg DP, Karim R, Dunussi-Joannopoulos K, Collins M, et al. Interleukin (IL)-22 and IL-17 are coexpressed by Th17 cells and cooperatively enhance expression of antimicrobial peptides. J Exp Med. 2006. 203(10):2271-9.
4. Trifari S, Kaplan CD, Tran EH, Crellin NK, Spits H. Identification of a human helper T cell population that has abundant production of interleukin 22 and is distinct from T(H)-17, T(H)1 and T(H)2 cells. Nat Immunol. 2009. 10(8):864-71.
5. Acosta-Rodriguez EV, Rivino L, Geginat J, Jarrossay D, Gattorno M, Lanzavecchia A, et al. Surface phenotype and antigenic specificity of human interleukin 17-producing T helper memory cells. Nat Immunol. 2007. 8(6):639-46.

## Claims

1. Interleukin-22 (IL-22) for use as a prognostic marker for bone erosion associated with rheumatoid arthritis.

2. IL-22 according to claim 1, wherein a concentration of IL-22 measured in the early stages of rheumatoid arthritis, which is increased as compared to the concentration of IL-22 in healthy controls, indicates an increased risk of developing bone erosions early, for example within 24 months, preferably within 12 months, more preferably within 10 months, even more preferably within 8 months after the initial clinical symptoms of rheumatoid arthritis.

3. IL-22 according to claim 2, wherein said increased concentration of IL-22 is measured within the first 6 months, preferably within the first 5 months, more preferably within the first 4 months, most preferably within the first 3 months after the initial clinical symptoms of rheumatoid arthritis.

4. IL-22 according to claim 2 or 3, wherein said concentration of IL-22 is increased by at least 75%, preferably by at least 100%, most preferably by at least 125%, as compared to the concentration of IL-22 in healthy controls.

5. Method of prognosing the development of bone erosion in a subject suffering from rheumatoid arthritis, said method comprising the steps of:
a) measuring the concentration of IL-22 in a sample derived from said subject; and
b) comparing said concentration of IL-22 with the concentration of IL-22 in healthy controls;
wherein said method is performed within the first 6 months, preferably within the first 5 months, more preferably within the first 4 months, most preferably within the first 3 months after the initial clinical symptoms of rheumatoid arthritis, and
wherein an increased concentration of IL-22 as compared to the concentration of IL-22 in healthy controls indicates an increased risk of developing bone erosions early, for example within 24 months, preferably within 12 months, more preferably within 10 months, even more preferably within 8 months after the initial clinical symptoms of rheumatoid arthritis.

6. Method according to claim 5, wherein said sample is selected from the group consisting of a blood sample, a serum sample and a plasma sample, wherein a serum sample is preferred.

7. Method according to claim 5 or 6, wherein said concentration of IL-22 is increased by at least 75%, preferably by at least 100%, most preferably by at least 125%, as compared to the concentration of IL-22 in healthy controls.

8. Interleukin-22 (IL-22) for use as a diagnostic marker for the progression of rheumatoid arthritis.

9. IL-22 according to claim 8, wherein the concentration of IL-22 correlates with the severity of synovitis and/or the number of tender and swollen joints, such that a higher concentration of IL-22 corresponds to more severe synovitis and/or a higher number of tender and swollen joints.

10. Method of diagnosing the progression of rheumatoid arthritis in a subject suffering from rheumatoid arthritis, said method comprising the step of measuring the concentration of IL-22 in a sample derived from said subject, wherein the concentration of IL-22 correlates with the severity of synovitis and/or the number of tender and swollen joints, such that a higher concentration of IL-22 corresponds to more severe synovitis and/or a higher number of tender and swollen joints.

11. Method according to claim 10, wherein said sample is selected from the group consisting of a blood sample, a serum sample and a plasma sample, wherein a serum sample is preferred.

12. Use of an IL-22-specific test kit for prognosing the development of bone erosion or diagnosing the progression of rheumatoid arthritis in a subject suffering from rheumatoid arthritis.
